# EUROPÄISCHE PATENTSCHRIFT

(11) **EP 3 801 364 B1**
(45) Veröffentlichungstag und Bekanntmachung des Hinweises auf die Patenterteilung: **06.12.2023**
(21) Anmeldenummer: 19721571.8
(22) Anmeldetag: 24.04.2019
(51) Int. Cl.: A61C 8/00, A61L 27/32

(54) **VERFAHREN ZUM HERSTELLEN EINES DENTALIMPLANTATS UND DERART HERGESTELLTES DENTALIMPLANTAT**
METHOD FOR PRODUCING A DENTAL IMPLANT AND DENTAL IMPLANT PRODUCED BY SAID METHOD
METHODE DE PRODUCTION D'UN IMPLANT DENTAIRE ET IMPLANT DENTAIRE PRODUIT PAR CETTE METHODE

(30) Priorität: 30.05.2018 DE 102018112935
(43) Veröffentlichungstag der Anmeldung: 14.04.2021
(73) Patentinhaber: ZV3 - Zircon Vision GmbH, 82515 Wolfratshausen (DE)
(72) Erfinder: FEITH, Johan, 82547 Eurasburg/Achmühle (DE)
(74) Vertreter: Meissner Bolte Partnerschaft mbB
(86) Internationale Anmeldenummer: PCT/EP2019/060518
(87) Internationale Veröffentlichungsnummer: WO 2019/228713

(56) Entgegenhaltungen:
- EP-A1- 2 316 374
- JP-B2- 2 893 253
- KR-A- 20030 078 480
- US-A- 5 934 287
- US-A1- 2010 010 632
- KAROLINE PARDUN ET AL: "Characterization of Wet Powder-Sprayed Zirconia/Calcium Phosphate Coating for Dental Implants : Mixed Surface Coatings with Firm Adhesion", CLINICAL IMPLANT DENTISTRY AND RELATED RESEARCH, Bd. 17, Nr. 1, 2015, Seiten 186-198, XP055605062, CA ISSN: 1523-0899, DOI: 10.1111/cid.12071

## Beschreibung

Die Erfindung betrifft ein Verfahren zum Herstellen eines Dentalimplantats gemäß Patentanspruch 1. Des Weiteren betrifft die Erfindung ein Dentalimplantat, das gemäß dem erfindungsgemäßen Verfahren hergestellt ist.

Es ist bekannt, Dentalimplantate, insbesondere Dentalimplantatbauteile, wie zum Beispiel Verankerungsstifte und/oder Abutments, aus einem derartigen Material zu fertigen, die im Zusammenhang mit einem optimalen Knochenwachstum stehen, d.h., dass die Dentalimplantatbauteile aus einem derartigen Material gefertigt werden, dass diese optimal in den Kieferknochen einwachsen können.

Aus dem Artikel "Characterization of wet powder-sprayed zirconia/calcium phosphate coating for dental implants: Mixed Surface Coatings with Firm Adhesion" von Karoline Pardun et al in CLINICAL IMPLANT DENTISTRY AND RELATED RESEARCH, BD. 17, Nr. 1, 2015, Seiten 186-198, ist es beispielsweise bekannt auf ein Dentalimplantat eine Besichtung aufzubringen. Insbesondere wird beschrieben, dass die Beschichtung aus einer Calciumphosphat/Zirconia-Mischung besteht. Es wird beschrieben, dass dieses Material mit einer Art von Spraypistole auf ein Muster aufgetragen wird. Es ist dabei zu beachten, dass mit einem Druck von 2 bar gearbeitet wird, wobei der Düsendurchmesser 0,8 mm beträgt. Des Weiteren ist es bekannt, die Oberflächen von Dentalimplantatbauteilen aufzurauen. Derartig aufgeraute Oberflächen verbessern ebenfalls die Osteogenese.

Es ist bekannt, derartiges Aufrauen mittels Sandstrahlen durchzuführen. Anschließend wird die Oberfläche aufwendig gereinigt, so dass Strahlmittel-Partikel von der die Osteogenese optimal fördernden Oberfläche größtenteils entfernt werden. In EP 2 316 374 A1 wird beispielsweise ein derartiger Aufrauprozess im Sinne eines Sandstrahlverfahrens beschrieben.

US 5 934 287 A betrifft wiederum ein Verfahren, zur Herstellung einer bioaktiven Oberfläche eines Dentalimplantats, wobei dort Bearbeitungsverfahren durchgeführt werden. In einem ersten Verfahrensschritt erfolgt das Aufrauen einer Oberfläche mittels eines Sandstrahlverfahrens. In einem weiteren Verfahrensschritt wird eine Beschichtung auf der aufgerauten Oberfläche aufgetragen.

Aus dem Vorgenannten wird klar, dass der bislang bekannte Stand der Technik hinsichtlich optimierter Verfahren zum Herstellen von Dentalimplantaten mehrere Nachteile aufweist.

Beispielsweise ist bereits bei der Formung des Dentalimplantatbauteils auf spezielle Materialien abzustellen, die unter Umständen teuer in der Produktion sind. Das Aufrauen von Oberflächen ist bislang mit dem Nachteil verbunden, dass aufwendige Reinigungsmaßnahmen notwendig werden.

Aus dem Vorgenannten ist es somit Aufgabe der vorliegenden Erfindung, ein weiterentwickeltes Verfahren zum Herstellen eines Dentalimplantates anzugeben, das die vorgenannten Nachteile des Standes der Technik überwindet.

Des Weiteren soll ein Dentalimplantat angegeben werden, das mittels eines erfindungsgemäßen Verfahrens hergestellt ist.

Erfindungsgemäß wird diese Aufgabe im Hinblick auf das Verfahren zum Herstellen eines Dentalimplantats durch Patentanspruch 1 gelöst.

Im Hinblick auf das Dentalimplantat wird diese Aufgabe durch den Gegenstand des Patentanspruches 6 gelöst.

Die Erfindung beruht auf dem Gedanken, ein Verfahren zum Herstellen eines Dentalimplantats anzugeben, das die folgenden Schritte umfasst:
a) Bereitstellen eines Dentalimplantat-Grundkörpers, insbesondere eines Verankerungsstift-Grundkörpers, wobei im Schritt a) ein Vorsintern/Vorbrennen des Dentalimplantat-Grundkörpers bei einer Vorsintertemperatur von 600 °C - 1.100 °C umfasst;
b) Strahlen mindestens eines Oberflächenabschnitts des Dentalimplantat-Grundkörpers, der mit einer das Knochenwachstum fördernden Oberfläche auszubilden ist, und zumindest abschnittsweises Aufbringen von Strahlmittel auf den Oberflächenabschnitt, wobei aufgrund des Strahlens im Sinne eines Sandstrahlens, der Oberflächenabschnitt mit einer Oberflächenrauigkeit mit einer maximalen Rautiefe Ra von 5 µm - 15 µm ausgebildet wird und zusätzlich das Strahlmittel aufgebracht wird;
c) höchstens teilweises Entfernen des Strahlmittels von dem Oberflächenabschnitt;
d) Sintern des Dentalimplantat-Grundkörpers zusammen mit dem auf dem Oberflächenabschnitt verbliebenen Strahlmittel, wobei als Strahlmittel ein Pulver verwendet wird, das Calciumphosphat-Partikel und Hydroxylapatit-Partikel aufweist.

Mit anderen Worten wird zunächst ein Grundkörper eines Dentalimplantats zur Verfügung gestellt. Bei dem Grundkörper kann es sich um den Grundkörper eines Verankerungsstiftes handeln.

Der Grundkörper kann beispielsweise als Oxidkeramik-Grünkörper, insbesondere als Zirkonoxid-Grünkörper, ausgebildet sein. Vorzugsweise kann dieser Grundkörper ein Übermaß zur Kompensation einer Schrumpfung während etwaiger Sinter- oder Brennvorgängen aufweisen.

Vor dem Schritt b) kann mindestens ein Oberflächenabschnitt bestimmt oder festgelegt werden, der mit einer, das Knochenwachstum fördernden Oberfläche auszubilden ist.

Aufgrund des erfindungsgemäßen Verfahrens ist es möglich, lediglich einen Abschnitt oder lediglich Abschnitte der Oberfläche des Dentalimplantat-Grundkörpers mit einer das Knochenwachstum fördernden Oberfläche auszubilden. Es ist aufgrund des zur Verfügung gestellten Verfahrens nicht notwendig, die gesamte Oberfläche des Dentalimplantats mit einer das Knochenwachstum fördernden Oberfläche auszubilden. Allerdings kann auch die gesamte Oberfläche des Dentalimplantats mit einer das Knochenwachstum fördernden Oberfläche ausgebildet werden.

Es kann beispielsweise die gesamte äußere Oberfläche des Grundkörpers des Dentalimplantats mit einer derartigen das Knochenwachstum fördernden Oberfläche ausgebildet werden.

Zur Ausbildung dieser, das Knochenwachstum fördernden Oberfläche wird der Oberflächenabschnitt gestrahlt. Aufgrund des Strahlens im Sinne eines Sandstrahlens, wird der Oberflächenabschnitt mit einer Oberflächenrauigkeit ausgebildet, die das Knochenwachstum, d.h. die Osteogenese, fördert.

Zusätzlich zur Ausbildung einer Oberflächenrauigkeit wird außerdem zumindest abschnittsweise auf dem zu bearbeitenden Oberflächenabschnitt ein Strahlmittel aufgebracht.

Das während des Strahlens des Oberflächenabschnitts aufgetragene Strahlmittel schlägt auf dem Oberflächenabschnitt ein. Das eingeschlagene Strahlmittel verbleibt, vorzugsweise größtenteils, besonders bevorzugt vollständig, auf der Oberfläche.

Im Schritt b) wird eine Oberfläche mit einer das Knochenwachstum fördernden Rauigkeit hergestellt, wobei zusätzlich Partikel des Strahlmittels als knochenwachstumsfördernde Oberfläche dienen.

Im Schritt c) wird das aufgebrachte Strahlmittel höchstens teilweise von dem Oberflächenabschnitt entfernt. Demnach ist es in Schritt c) vorzugsweise nicht vorgesehen, gezielte Reinigungsschritte zum Entfernen des Strahlmittels von dem bearbeiteten Oberflächenabschnitt vorzunehmen. Reinigungsschritte wie Waschschritte oder Ultraschall-Reinigungen können somit zumindest teilweise entfallen. Ein Kanal eines Verankerungsstiftes sollte beispielsweise nach dem Strahlvorgang trotzdem gereinigt werden. Diese Reinigung kann mit Hilfe eines Ultraschallbads erfolgen.

Vorzugsweise werden im Schritt c) lediglich Schmutz- und/oder Staubpartikel entfernt, jedoch nicht das Strahlmittel. Des Weiteren ist es möglich, dass im Schritt c) eine derartige Reinigung erfolgt, sodass lose Strahlmittel-Partikel entfernt werden. Als lose Strahlmittel-Partikel sind insbesondere derartige Strahlmittel-Partikel zu verstehen, die keine Verbindung mit dem zu bearbeitenden Oberflächenabschnitt und/oder mit anderen Strahlmittel-Partikeln eingegangen sind.

Im Schritt d) erfolgt ein Sintern des Dentalimplantat-Grundkörpers zusammen mit dem auf dem Oberflächenabschnitt verbliebenen Strahlmittel. Der Dentalimplantat-Grundkörper wird insbesondere zusammen mit Strahlmittel-Partikeln gesintert, die bereits vor dem Sintern mit der zu bearbeitenden Oberfläche und/oder mit weiteren Strahlmittel-Partikeln verbunden sind. Diese Verbindung wird aufgrund des im Schritt b) durchgeführten Strahlens erzeugt.

Es ist möglich, dass das Dentalimplantat im Schritt d) bei einer Temperatur von 1.300 °C - 1.600 °C, insbesondere von 1.400 °C - 1.550 °C, gesintert/gebrannt wird.

Dabei wir in einem Sinterofen vorzugsweise zunächst ein langsamer Temperaturanstieg gewählt. Vorzugsweise beträgt der Temperaturanstieg ca. 100 °C pro Stunde. Sobald die Endtemperatur erreicht ist, sollte diese Temperatur für ca. 0,5 - 4,0 Stunden, insbesondere für 1,0 - 3,0 Stunden, besonders bevorzugt für ca. 2,0 Stunden, gehalten werden. Anschließend kann die Temperaturzufuhr abgestellt werden, wobei das Dentalimplantat bis zur vollständigen Abkühlung im Sinterofen positioniert bleibt. Alternativ ist es möglich, dass die Temperatur im Sinterofen langsam abfällt.

In diesem Schritt findet somit das abschließende Sintern bzw. Brennen des Dentalimplantatbauteils statt. Anschließend kann ein verwendbares Dentalimplantat, insbesondere ein verwendbarer Verankerungsstift, vorliegen. Der Materialschrumpf nach dem finalen Sintern, bzw. nach Durchführen des Verfahrensschritts d) beträgt 21 % - 27 %, besonders bevorzugt 25 %. Dieser Materialschrumpf ist im Verfahrensschritt b) zu beachten, da die nach Schritt b) erzielte Rauigkeit des Oberflächenabschnitts aufgrund des Materialschrumpfs abnimmt.

Das Strahlmittel wird im Schritt b) vorzugsweise mit einem Druck von 0,1 bar - 10,0 bar, insbesondere von 1,0 bar - 5,0 bar, besonders bevorzugt von 1,5 bar - 2,5 bar auf den Oberflächenabschnitt aufgebracht.

Vorzugsweise wird zum Auftragen des Strahlmittels eine Düse mit einem Düsenquerschnitt von 0,6 mm - 1,5 mm verwendet.

Im Schritt a) wird ein derartiger Dentalimplantat-Grundkörper bereitgestellt, der vorgesintert/vorgebrannt ist. Mit anderen Worten umfasst der Schritt a) ein Vorsintern/Vorbrennen des Dentalimplantat-Grundkörpers.

Der Dentalimplantatbauteil-Grundkörper wird bei einer Temperatur von 600 °C - 1.100 °C, insbesondere von 700 °C - 900 °C, vorgesintert/vorgebrannt. Dabei handelt es sich um niedrige Vorsintertemperaturen. Der Grundkörper des Dentalimplantats ist somit im Vergleich zu Grundkörpern aus dem Stand der Technik weicher, so dass das Strahlmittel bzw. Partikel des Strahlmittels einfacher auf den Oberflächenabschnitt aufgetragen werden kann/können.

Der Druck, mit dem das Strahlmittel im Schritt b) auf den Oberflächenabschnitt aufgebracht wird, ist auf Grundlage des bereitgestellten Dentalimplantat-Grundkörpers zu wählen. Je weicher der zu strahlende Oberflächenabschnitt ist, desto geringer kann der Druck bzw. Strahldruck gewählt werden. Die Härte des Oberflächenabschnitts steht im Zusammenhang mit der gewählten Vorsintertemperatur. Bei einer Vorsintertemperatur von 800 °C ist beispielsweise ein Strahldruck von ca. 1,0 bar zu wählen. Bei einer Vorsintertemperatur von 1.000 °C ist beispielsweise ein Strahldruck von ca. 2,0 bar zu wählen.

Bei dem Strahlmittel handelt es sich um eine Calciumphosphat-Verbindung in Pulverform und eine Hydroxylapatit-Verbindung in Pulverform. Erfindungsgemäß wird demnach ein Strahlmittel verwendet, das Calciumphosphat-Pulver und Hydroxylapatit-Pulver umfasst. Des Weiteren ist es möglich, dass das Strahlmittel aus Calciumphosphat-Pulver und Hydroxylapatit-Pulver besteht.

Es ist möglich, dass vor dem Schritt b) auf den Grundkörper des Dentalimplantats eine Maske aufgebracht wird, so dass die zu strahlenden Oberflächenabschnitte von den nicht zu strahlenden Oberflächenabschnitten klar getrennt sind.

Des Weiteren ist es möglich, eine Strahldüse derart zu positionieren und während des Strahlvorganges zu verfahren, dass lediglich die zuvor bestimmten Oberflächenabschnitte gestrahlt bzw. gesandstrahlt werden.

Vorzugsweise wird die das Knochenwachstum fördernde Oberfläche eines Dentalimplantats vollständig vor dem abschließenden Brennen/Sintern hergestellt.

Das erfindungsgemäße Verfahren hat den Vorteil, dass keine aufwendigen Reinigungsvorgänge notwendig sind, obwohl ein Strahlen, im Sinne eines Sandstrahlens, einer Oberfläche durchgeführt wird.

Ein weiterer, insbesondere nebengeordneter, Aspekt der Erfindung betrifft ein Dentalimplant, insbesondere einen Verankerungsstift, das/der nach einem erfindungsgemäßen Verfahren hergestellt ist.

Das Dentalimplantat weist mindestens einen Oberflächenabschnitt auf, der Calciumphosphat-Partikel und Hydroxylapatit-Partikel aufweist. Die Calciumphosphat-Partikel und die Hydroxylapatit-Partikel sind auf mindestens einem Oberflächenabschnitt des Dentalimplantats ausgebildet, der als das Knochenwachstum fördernder Oberflächenabschnitt ausgebildet ist. Vorzugsweise ist auf dem Oberflächenabschnitt des Dentalimplantats eine Schicht ausgebildet, die Calciumphosphat-Verbindungen und Hydroxylapatit-Verbindungen aufweist.

Des Weiteren weist dieser Oberflächenabschnitt eine Rauigkeit auf, die ebenfalls fördernd für das Knochenwachstum, d.h. für eine Osteogenese, ist. Die Rauigkeit des Oberflächenabschnitts ist mit einer maximalen Rautiefe Ra von 5 µm - 15 µm ausgebildet.

Gemäß einer Ausführungsform der Erfindung ist es möglich, dass der Verankerungsstift und das Abutment einstückig ausgebildet sind. Dadurch wird ein in ästhetischer Hinsicht optimiertes Dentalimplantatbauteil zur Verfügung gestellt. Außerdem ist die Haltbarkeit eines derartigen Dentalimplantatbauteils erhöht.

Bei der beschriebenen einstückigen Ausbildung eines Verankerungsstifts mit dem Abutment kann das Dentalimplantatbauteil auf der gesamten Oberfläche eine das Knochenwachstum fördernde Oberfläche aufweisen.

Alternativ ist es möglich, dass ein Abschnitt der Oberfläche, der am Abutment ausgebildet ist, keine derartige Oberfläche aufweist, die das Knochenwachstum fördert. Vielmehr ist es vorgesehen, dass ein Oberflächenabschnitt des Abutments eine relativ geringe Rauigkeit aufweist, so dass ein Kronenteil einfach auf das Abutment aufgesetzt bzw. aufgeschoben werden kann.

Ein weiterer Aspekt der Erfindung betrifft ein Strahlmittel zur Verwendung in dem erfindungsgemäßen, Verfahren zum Herstellen eines Dentalimplantats.

Erfindungsgemäß handelt es sich bei dem Strahlmittel um ein Pulver, das eine Calciumphosphat-Verbindung und eine Hydroxylapatit-Verbindung umfasst.

Bei dem Strahlmittel kann es sich demnach um Ca₁₀(PO₄)₆(OH)₂ / Ca₃(PO₄)₂ handeln.

In einer besonders bevorzugten Ausführungsform der Erfindung umfasst das Pulver mindestens 5 %, insbesondere mindestens 10 %, Calciumphosphat und/oder mindestens 30 %, insbesondere mindestens 50 %, Calciumphosphat-Verbindungen.

Vorzugweise weist das Pulver höchstens 30 %, insbesondere höchstens 20%, Hydroxylapatit-Verbindungen auf.

In einer besonders bevorzugten Ausführungsform der Erfindung weisen die Partikel eine Größe von 25 µm - 150 µm, insbesondere von 50 µm - 100 µm, auf. Insbesondere sind die Partikel eines zu verwendenden Pulvers unregelmäßig geformt. Aufgrund dessen kann ein Dentalimplantat hergestellt werden, das auf einem Oberflächenabschnitt die beschriebenen Partikel aufweist, wobei gleichzeitig eine bestimmte Oberflächenrauigkeit eingestellt werden kann.

Nachfolgend wird die Erfindung anhand einer Abbildung näher erläutert.

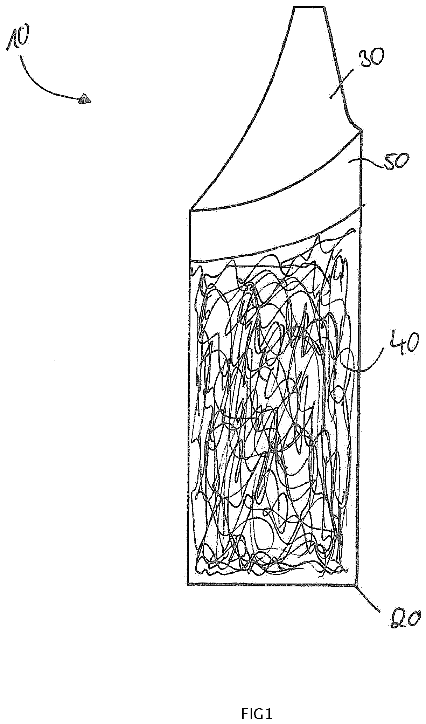

In der Figur wird ein Dentalimplantatbauteil 10 dargestellt, welches gemäß einem erfindungsgemäßen Verfahren hergestellt wurde.

Das Dentalimplantatbauteil 10 umfasst einen Verankerungsstift 20 sowie ein Abutment 30. Der Verankerungsstift 20 weist dabei einen Oberflächenabschnitt 40 auf, der mit einer das Knochenwachstum fördernden Oberfläche ausgebildet ist. Es ist zu erkennen, dass es sich dabei um lediglich einen Teilabschnitt der vollständigen Oberfläche des Verankerungsstiftes 20 handelt. Ein zweiter Oberflächenabschnitt 50 des Verankerungsstiftes 20, der im implantierten Zustand am Zahnfleisch anliegt, wird nicht mit einer das Knochenwachstum fördernden Oberfläche ausgebildet.

Der Oberflächenabschnitt 40 wird dabei im Rahmen eines Strahlvorganges, im Sinne eines Sandstrahlvorganges, hergestellt. Dabei wird ein Strahlmittel, erfindungsgemäß ein Pulver, das Calciumphosphat und Hydroxylapatit umfasst, auf den Oberflächenabschnitt 40 aufgebracht.

Das Strahlmittel wird anschließend nicht oder zumindest nicht vollständig entfernt, so dass das Strahlmittel bzw. die Partikel des Strahlmittels einen Teil der das Knochenwachstum fördernden Oberfläche darstellt/darstellen.

Zwischen den einzelnen Partikeln können Leerstellen ausgebildet sein, so dass der Oberflächenabschnitt 40 auch eine entsprechende Rauigkeit aufweist.

Zusammenfassend kann mit Hilfe eines vereinfachten Verfahrens ein Dentalimplantatbauteil zur Verfügung gestellt werden, das zumindest abschnittsweise eine verbesserte Oberfläche hinsichtlich der Knochenwachstumsförderung, d.h. der Osteogenese, aufweist.

### Bezugszeichenliste

- 10: Dentalimplantatbauteil
- 20: Verankerungsstift
- 30: Abutment
- 40: erster Oberflächenabschnitt
- 50: zweiter Oberflächenabschnitt

## Patentansprüche

1. Verfahren zum Herstellen eines Dentalimplantats (20), umfassend die Schritte:
a) Bereitstellen eines Dentalimplantat-Grundkörpers, insbesondere eines Verankerungsstift-Grundkörpers, wobei Schritt a) ein Vorsintern/Vorbrennen des Dentalimplantat-Grundkörpers bei einer Vorsintertemperatur von 600 °C - 1.100 °C umfasst;
b) Strahlen mindestens eines Oberflächenabschnitts (40) des Dentalimplantat-Grundkörpers, der mit einer das Knochenwachstum fördernden Oberfläche auszubilden ist, und zumindest abschnittsweises Aufbringen von Strahlmittel auf den Oberflächenabschnitt, wobei aufgrund des Strahlens im Sinne eines Sandstrahlens, der Oberflächenabschnitt mit einer Oberflächenrauigkeit mit einer maximalen Rautiefe Ra von 5 µm - 15 µm ausgebildet wird und zusätzlich das Strahlmittel aufgebracht wird;
c) höchstens teilweises Entfernen des Strahlmittels von dem Oberflächenabschnitt (40);
d) Sintern des Dentalimplantat-Grundkörpers zusammen mit dem auf dem Oberflächenabschnitt (40) verbliebenen Strahlmittel,
wobei
als Strahlmittel ein Pulver verwendet wird, das Calciumphosphat-Partikel und Hydroxylapatit-Partikel aufweist.

2. Verfahren nach Anspruch 1,
**dadurch gekennzeichnet, dass**
das Strahlmittel mit einem Druck von 0,1 bar - 10,0 bar, insbesondere von 1,0 bar - 5,0 bar, besonders bevorzugt von 1,5 bar - 2,5 bar, auf den Oberflächenabschnitt (40) aufgebracht wird.

3. Verfahren nach Anspruch 1 oder 2,
**dadurch gekennzeichnet, dass**
der Dentalimplantat-Grundkörper bei einer Temperatur von 700 °C - 900 °C vorgesintert/vorgebrannt wird.

4. Verfahren nach einem der vorangegangenen Ansprüche,
**dadurch gekennzeichnet, dass**
der Dentalimplantat-Grundkörper im Schritt d) bei einer Temperatur von 1.300 °C - 1.600 °C, insbesondere von 1.400 °C - 1.550 °C, gesintert/gebrannt wird.

5. Verfahren nach einem der vorangegangenen Ansprüche,
**dadurch gekennzeichnet, dass**
im Schritt c) eine Reinigung erfolgt, derart, dass lose Strahlmittel-Partikel entfernt werden.

6. Dentalimplantat (20), hergestellt nach einem Verfahren nach einem der Ansprüche 1 bis 5.

## Claims

1. Method of manufacturing a dental implant (20),
comprising the steps:
a) provision of a dental implant base body, in particular an anchoring pin base body, wherein step a) comprises a presintering/prefiring of the dental implant base body at a presintering temperature of 600°C-1,100°C;
b) abrasive blasting of at least one surface portion (40) of the dental implant base body, which is to be configured with a surface that promotes bone growth, and at least regional application of abrasive blasting agent to the surface portion, wherein, due to the abrasive blasting in the sense of sandblasting, the surface portion is configured with a surface roughness with a maximum roughness depth Ra of 5 µm - 15 µm and the abrasive blasting is additionally applied;
c) at most partial removal of the abrasive blasting agent from the surface portion (40);
d) sintering of the dental implant base body together with the abrasive blasting agent remaining on the surface portion (40), wherein a powder having calcium phosphate particles and hydroxyapatite particles is used as the abrasive blasting.

2. Method according to claim 1,
**characterized in that**
the abrasive blasting agent is applied to the surface portion (40) at a pressure of 0.1 bar-10.0 bar, in particular of 1.0 bar-5.0 bar, particularly preferably of 1.5 bar-2.5 bar.

3. Method according to claim 1 or 2,
**characterized in that**
the dental implant base body is sintered/fired at a temperature of 700°C-900°C.

4. Method according to one of the preceding claims,
**characterized in that**
the dental implant base body is sintered/fired in step d) at a temperature of 1,300°C-1,600°C, in particular 1,400°C-1,550°C.

5. Method according to one of the preceding claims,
**characterized in that**
in step c) cleaning is carried out in such a way that loose abrasive blasting agent particles are removed.

6. Dental implant (20) produced by a method according to one of claims 1 to 5.

## Revendications

1. Procédé de fabrication d'un implant dentaire (20), comprenant les étapes suivantes :
a) fourniture d'un corps de base d'implant dentaire, en particulier d'un corps de base de broche d'ancrage, dans lequel l'étape a) comprend un préfrittage/une précuisson du corps de base d'implant dentaire à une température de préfrittage de 600 °C à 1 100 °C ;
b) grenaillage d'au moins une partie de surface (40) du corps de base d'implant dentaire qui doit être formée avec une surface favorisant la croissance osseuse, et application d'un agent de grenaillage au moins par endroits sur la partie de surface, dans lequel, en raison du grenaillage au sens d'un sablage, la partie de surface est formée avec une rugosité de surface ayant une profondeur de rugosité maximale Ra de 5 µm à 15 µm et l'agent de grenaillage est en outre appliqué ;
c) élimination tout au plus partielle de l'agent de grenaillage de la partie de surface (40) ;
d) frittage du corps de base d'implant dentaire avec l'agent de grenaillage restant sur la partie de surface (40),
dans lequel on utilise comme agent de grenaillage une poudre qui présente des particules de phosphate de calcium et des particules d'hydroxyapatite.

2. Procédé selon la revendication 1,
**caractérisé en ce que**
l'agent de grenaillage est appliqué sur la partie de surface (40) avec une pression de 0,1 bar à 10,0 bars, en particulier de 1,0 bar à 5,0 bars, de manière particulièrement préférée de 1,5 bar à 2,5 bars.

3. Procédé selon la revendication 1 ou 2,
**caractérisé en ce que**
le corps de base d'implant dentaire est préfritté/précuit à une température de 700 °C à 900 °C.

4. Procédé selon l'une des revendications précédentes,
**caractérisé en ce que**
le corps de base d'implant dentaire est fritté/cuit à l'étape d) à une température de 1 300 °C à 1 600 °C, en particulier de 1 400 °C à 1 550 °C.

5. Procédé selon l'une des revendications précédentes,
**caractérisé en ce que**,
à l'étape c), un nettoyage est effectué de telle sorte que les particules d'agent de grenaillage non adhérentes soient éliminées.

6. Implant dentaire (20) fabriqué selon un procédé selon l'une des revendications 1 à 5.
